# EUROPEAN PATENT APPLICATION

(11) **EP 2 522 378 A1**
(43) Date of publication of application: **14.11.2012**
(21) Application number: 11165610.4
(22) Date of filing: 11.05.2011
(51) Int. Cl.: A61M 5/00, B65D 83/02, B65D 85/24, B65D 83/04

(54) **Needle assembly storage device**

(71) Applicant: Sanofi-Aventis Deutschland GmbH, 65929 Frankfurt (DE)
(72) Inventor: The designation of the inventor has not yet been filed

(57) **Abstract**

Described is a needle assembly storage device (1) comprising a case (2) having an opening (4), an array (5) of needle assembly storage compartments (6) disposed in the case (2), a transport mechanism coupled to a plurality of the needle assembly storage compartments (6), and a handle (10) coupled to the case (2). When the handle (10) is moved from a first position to a second position, the transport mechanism moves the plurality of the needle assembly storage compartments (6) within the case (2) to align a given one of the needle assembly storage compartments (6) with the opening (4).

## Description

### Technical Field

The invention relates to a needle assembly storage device for storing needle assemblies used with injection devices, such as pen injectors, autoinjectors, etc.

### Background of the Invention

Patients suffering from diseases like diabetes have to frequently self-administer injections. Injection devices like auto-injectors or pen injectors have been developed to facilitate self-administering injections. Typically, such injection devices, which are embodied in disposable and re-usable forms, are fitted with sterile injection needle assemblies to minimize the risk of infections. However, such needle assemblies generally come packaged in a box, which is inconvenient for the patient to transport. To solve this problem, needle assembly storage devices were developed. A conventional needle assembly storage device contains a plurality of injection needle assemblies arranged in a portable container. Such conventional needle assembly storage devices are discussed in US 7,134,550 and WO 2009/136193.

However, there remains a need for needle assembly storage devices which provide for portability of needle assemblies in a convenient manner and allow for disposing of used needle assemblies.

### Summary of the Invention

It is an object of the present invention to provide an improved device for storing and disposing needle assemblies.

In an exemplary embodiment, the present invention is a needle assembly storage device comprising a case having an opening, an array of needle assembly storage compartments disposed in the case, a transport mechanism coupled to a plurality of the needle assembly storage compartments, and a handle coupled to the case. When the handle is moved from a first position to a second position, the transport mechanism moves the plurality of the needle assembly storage compartments within the case to align a given one of the needle assembly storage compartments with the opening.

In an exemplary embodiment, the case comprises a first portion detachably coupled to a second portion.

In an exemplary embodiment, the handle may be one of rotatably and hingedly mounted to the case. The handle may include a cover portion, and when the handle is in the first position the cover portion may covers the opening. The handle may include a hole, and when the handle is in the second position the hole may be aligned with the opening. The handle may be biased in the first position.

In an exemplary embodiment, the transport mechanism includes a transport wheel having at least one section, and each of the at least one sections engages a given one of the needle assembly storage compartments. The transport wheel may rotate a predetermined step distance when the handle is moved between the first position and the second position. The transport mechanism may include an axle coupled to the handle and the transport wheel, a limiting member coupled to the axle and a stop. When the handle is moved between the first position and the second position, the axel may rotate and the limiting member may abut the stop.

In an exemplary embodiment, the handle may be disc-shaped and have a plurality of openings.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows a perspective view of a needle assembly storage device with a handle in a first handle position in an exemplary embodiment according to the present invention,
- Figure 2: shows a perspective view of a needle assembly storage device with a handle in a second handle position in an exemplary embodiment according to the present invention,
- Figure 3: shows a perspective view of a needle assembly storage device with an opened case and one storage compartment in an exemplary embodiment according to the present invention,
- Figure 4: shows a perspective view of an of needle assembly storage compartments in an exemplary embodiment according to the present invention,
- Figure 5: shows a perspective view of a portion of a needle assembly storage device with an opened case in an exemplary embodiment according to the present invention, and
- Figure 6: shows a perspective view of a needle assembly storage device with an opened case and a disc in an exemplary embodiment according to the present invention.

Corresponding parts are marked with the same reference symbols in all figures.

### Detailed Description

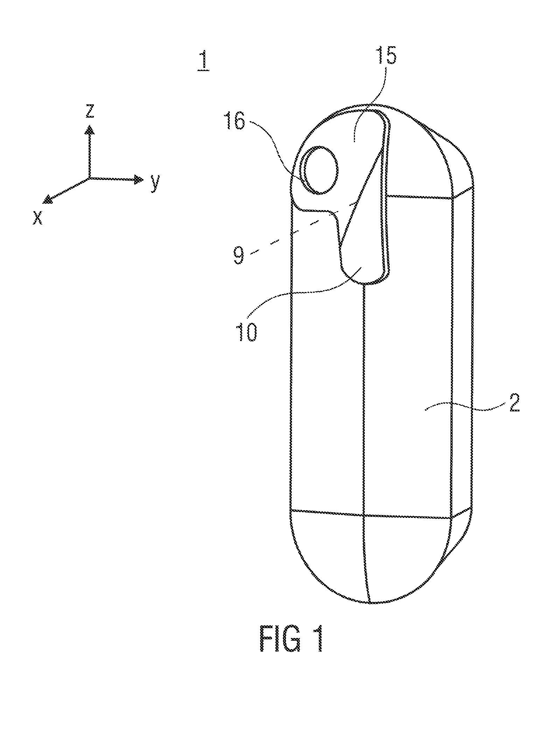

Figures 1 to 5 show an exemplary embodiment of a needle assembly storage device 1 according to the present invention. In an exemplary embodiment, the needle assembly storage device 1 comprises a case 2 with a cavity 3 formed therein for containing an array 5 of needle assembly storage compartments 6.

As shown in the exemplary embodiment of Figure 1, the case 2 may have an elongate (ellipsoidal or rectangular) shape having a length substantially equally to one half of the array 5 and a width substantially equal to a sum of widths of two needle assembly storage compartments 6. The case 2 may comprise a first portion detachably attached to a second portion. For example, when all of the needle assemblies in the storage device 1 have been used, the case 2 may be opened, exposing the cavity 3, to insert an array 5 of needle assembly storage compartments 6 containing unused needle assemblies. A lock may be disposed on the case 2 to lock the first and second portions in a closed position.

In an exemplary embodiment, a handle 10 may be disposed on the case 2 as shown in Figure 1. The handle 10 may be rotatably mounted (e.g., on an axis 9) or hingedly mounted (e.g., as a switch) on the case 2 and used to provide access to the needle assemblies and advance the array 5 within the case 2.

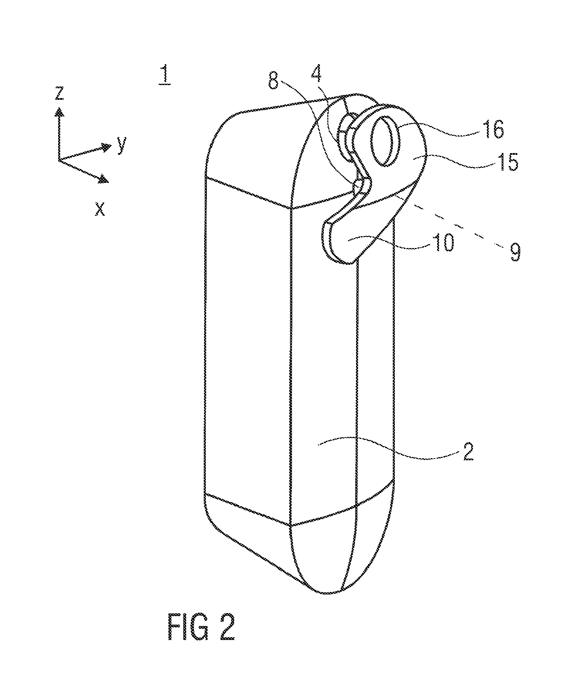

The handle 10 may be moveable between a first position (Figure 1) and a second position (Figure 2). In the first position, a cover portion 15 of the handle 10 may cover an opening 4 formed in the case 2. In the second position, the cover portion 15 may be displaced from the opening 4. In an exemplary embodiment, the handle 10 may include a hole which may be aligned with the opening when the handle 10 is in the second position. The hole 16 and the opening 4 may be adapted to receive a distal end of an injection device (e.g., pen injector, autoinjector, etc.), and the hole 16 and/or the opening 4 may guide the distal end of the injection device into a needle assembly such that a longitudinal axis of the injection device aligns with a longitudinal axis of the needle assembly. When an opening 12 of a given one of the needle assembly storage compartments 6 is aligned with the opening 4 and the handle 10 is in the second position, the injection device can be inserted to engage or disengage a needle assembly (allowing a needle 11 of the needle assembly to pierce a septum of a cartridge in the injection device). The handle 10 may be biased in the first position (e.g., by a spring). In another exemplary embodiment, the handle 10 may be manually movable between the first and second positions.

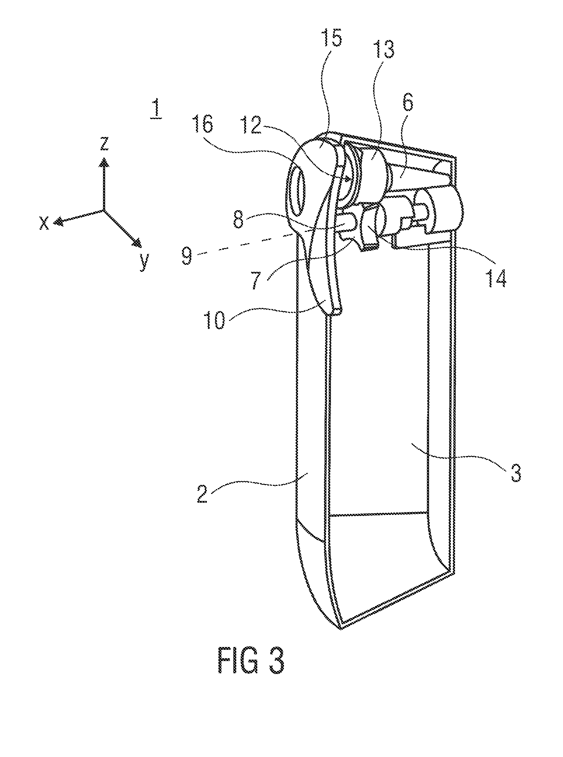

Figure 3 shows an exemplary embodiment of a transport mechanism for advancing the array 5 of the needle assembly storage compartments 6 within the case 2. In an exemplary embodiment, the transport mechanism includes an axle 8 which rotates in conjunction with the movement of the handle 10. A transport wheel 7 may be coupled to the axle 8. In an exemplary embodiment, the transport wheel 7 includes a plurality of sections 14, each adapted to engage a needle assembly storage compartment 6. The section 14 may have a concave shape for at least partially encircling a cylindrical outer surface 13 of the needle assembly storage compartment 6. In an exemplary embodiment, each time the handle 10 is moved into the second position, the axle 8 rotates and the transport wheel 7 advances the array 5 a step distance substantially equal to a width of a needle assembly storage compartment 6, thereby aligning a needle assembly storage compartment 6 with the opening 4. The transport wheel 7 may advance the array 5 by moving the needle assembly storage compartments 6 engaged by the sections 14 by the step distance.

In another exemplary embodiment, the transport wheel 7 rotates when the handle 10 is moved from the second position to the first position. For example, the user may move the handle 10 to the second position, engage an unused needle assembly via the opening 4, administer an injection, insert the used needle assembly into the same needle assembly storage compartment via the opening, and return the handle to the first position. When the handle 10 is moved from the second position to the first position, the transport wheel 7 rotates and advances the array 5 of the needle assembly storage compartments 6 such that when the handle 10 is subsequently moved to the second position from the first position, a needle assembly storage compartment 6 with an unused needle assembly is aligned with the opening 4.

In another exemplary embodiment, the first and second positions of the handle 10 may be as shown in Figures 1 and 2. However, movement to the second position may not rotate the transport wheel 7. Thus, the first position may be used to cover the opening 4 and the second position may be used to expose a needle assembly storage compartment 6 underlying the opening 4. Movement of the handle 10 to a third position may be utilized to rotate the transport wheel 7 and advance the array 5. In this exemplary embodiment, the user may move the handle 10 to the second position, engage an unused needle assembly via the opening 4, administer an injection, insert the used needle assembly into the same needle assembly storage compartment via the opening, and return the handle to the first position. When an unused needle assembly is needed, the handle 10 may be moved to the third position.

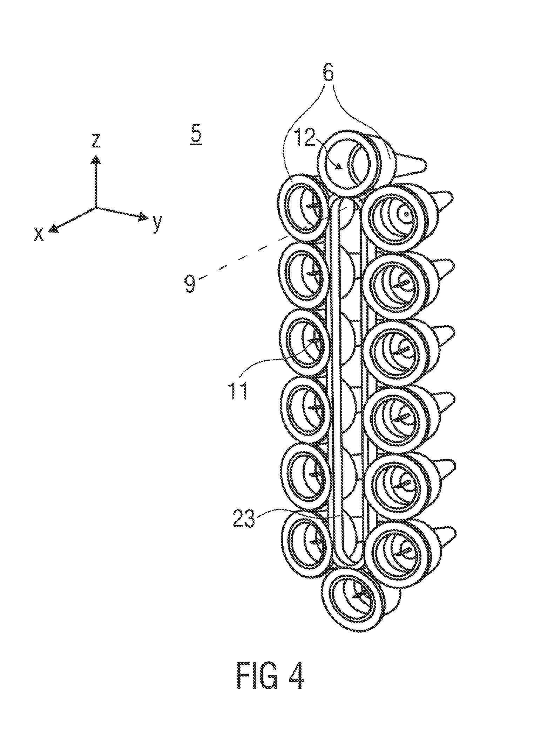

Figure 4 shows an exemplary embodiment of the array 5 of the needle assembly storage compartments 6. The array 5 may be a flexible chain of the needle assembly storage compartments 6, wherein consecutive needle assembly storage compartments 6 may be connected by a hinge. The array 5 may be produced by in a single injection moulding process. To form a loop, opposing ends of the array 5 may be coupled (e.g., via adhesive, welding, mechanical connection, etc.). The array 5 may be arranged around a track 23 formed in the case 2 and/or attached to the array 5. The track 23 may have a first end adjacent the axle 8 and a second end at an end of the case 2 opposite the axle 8. The needle assemblies and the needle assembly storage compartments 6 may include locking features to prevent reuse of a used needle assembly. The locking features may include, but are not limited to, those described in EP11164285.6, the entirety of which is incorporated herein by reference.

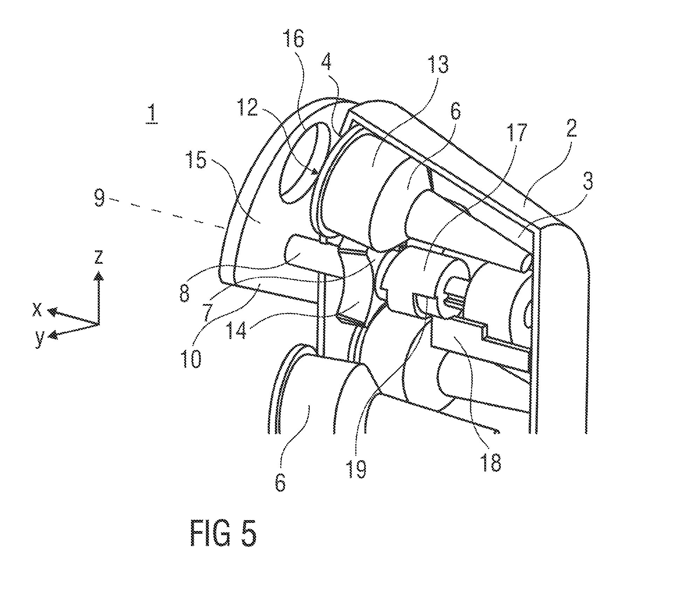

Figure 5 shows an exemplary embodiment of the transport mechanism according to the present invention. In an exemplary embodiment, the axle 8 is coupled to the handle 10 such that when the handle 10 is moved from the first position to the second position, the axle 8 rotates. The transport wheel 7 may be coupled to the axle 8 such that rotation of the axle 8 results in a corresponding rotation of the transport wheel 7. A limiting member 17 may also be coupled to, and rotate in conjunction with, the axle 8. In an exemplary embodiment, the limiting member 17 may include an abutment face 19 which abuts a stop 18 when the axle 8 has rotated a rotational distance equal to the step distance. In an exemplary embodiment, the predetermined rotational distance is configured such that, when the abutment face 19 abuts the stop 18, the hole 16 in the handle 10 is aligned with the opening 4. A spring (not shown) may be coupled to the axle 8 to bias the handle 10 in one of the positions.

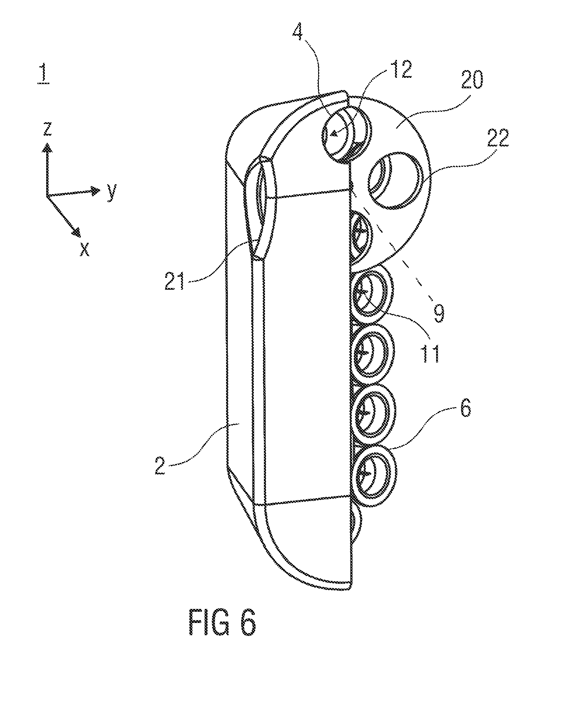

Figure 6 shows another exemplary embodiment of the handle 10. In the exemplary embodiment shown in Figure 6, the handle 10 is formed as a disc 20 which includes a plurality of openings 22 that may be aligned with the opening 4 in the case 2 to provide access to the needle assembly storage compartments 6. The disc 20 may be at least partially encased within the case 2 or entirely external to the case 2. If the disc 20 is at least partially encased within the case 2, an opening 21 may be formed in the case 2 to expose a portion of the disc 20 to enable manual actuation. The disc 20 may be coupled to the transport mechanism as described above to advance the array 5 within the case 2. Cover portions may be disposed between consecutive openings 22 in the disc 20 such that the opening 4 may be covered.

Those of skill in the art will understand the modifications (additions and/or removals) of various components of the device and/or system and embodiment described herein may be made without departing from the full scope and spirit of the present invention, which encompass such modifications and any and all equivalents thereof.

### List of References

- 1: needle assembly storage device
- 2: case
- 3: cavity
- 4: opening of case
- 5: array
- 6: needle assembly storage compartment
- 7: transport wheel
- 8: axle
- 9: rotation axis
- 10: handle
- 11: needle
- 12: opening of needle assembly storage compartment
- 13: cylindrical outer surface
- 14: section
- 15: cover portion
- 16: opening of handle
- 17: limiting member
- 18: stop
- 19: abutment face
- 20: disc
- 21: opening for disc
- 22: opening on disc
- 23: track
- x,y,z: direction

## Claims

1. A needle assembly storage device (1) comprising:
a case (2) having an opening (4);
an array (5) of needle assembly storage compartments (6) disposed in the case (2);
a transport mechanism coupled to a plurality of the needle assembly storage compartments (6);
a handle (10) coupled to the case (2), wherein when the handle (10) is moved from a first position to a second position, the transport mechanism moves the plurality of the needle assembly storage compartments (6) within the case (2) to align a given one of the needle assembly storage compartments (6) with the opening (4).

2. The needle assembly storage device (1) according to claim 1, wherein the case (2) comprises:
a first portion detachably coupled to a second portion.

3. The needle assembly storage device (1) according to any one of the previous claims, wherein the handle (10) is one of rotatably and hingedly mounted to the case (2).

4. The needle assembly storage device (1) according to any one of the previous claims, wherein the handle (10) includes a cover portion (15), and when the handle (10) is in the first position the cover portion (15) covers the opening (4).

5. The needle assembly storage device (1) according to any one of the previous claims, wherein the handle (10) includes a hole (16), and when the handle (10) is in the second position the hole (16) is aligned with the opening (4).

6. The needle assembly storage device (1) according to any one of the previous claims, wherein the handle (10) is biased in the first position.

7. The needle assembly storage device (1) according to any one of the previous claims, wherein the transport mechanism includes:
a transport wheel (7) having at least one section (14), wherein each of the at least one sections (14) engages a given one of the needle assembly storage compartments (6).

8. The needle assembly storage device (1) according to claim 7, wherein the transport wheel (7) rotates a predetermined step distance when the handle (10) is moved between the first position and the second position.

9. The needle assembly storage device (1) according to claim 7, wherein the transport mechanism includes:
an axle (8) coupled to the handle (10) and the transport wheel (7);
a limiting member (17) coupled to the axle (8); and
a stop (18),
wherein when the handle (10) is moved between the first position and the second position, the axel (8) rotates and the limiting member (17) abuts the stop (18).

10. The needle assembly storage device (1) according to any one of the previous claims, wherein the handle (10) is a disc (20).

11. The needle assembly storage device (1) according to claim 10, wherein the disc (20) includes a plurality of openings (22).
